# EUROPEAN PATENT APPLICATION

(11) **EP 4 803 010 A1**
(43) Date of publication of application: **09.09.2026**
(21) Application number: 26161671.8
(22) Date of filing: 02.03.2026
(51) Int. Cl.: A61B 8/00, G01S 7/52, G01S 15/89

(54) **ULTRASOUND DIAGNOSTIC APPARATUS, TRANSMISSION PATTERN DETERMINATION METHOD, AND TRANSMISSION PATTERN DETERMINATION PROGRAM**

(30) Priority: 03.03.2025 JP 2025032820
(71) Applicant: FUJIFILM Corporation, Tokyo 106-8620 (JP)
(72) Inventor: TANAKA, Tomohiko, Tokyo (JP); SHIMIZU, Motochika, Tokyo (JP); YAMADA, Tetsuya, Tokyo (JP); FUJITA, Kosuke, Tokyo (JP)
(74) Representative: Parker, Andrew James

(57) **Abstract**

An object of the present disclosure is to reduce the number of times of transmitting ultrasound pulses while maintaining Doppler measurement performance for an ultrasound diagnostic apparatus.

An intermittent transmission pattern determination method includes obtaining reference measurement information for reference transmission and intermittent measurement information for intermittent transmission, and obtaining error evaluation information representing a difference between the reference measurement information and the intermittent measurement information. The reference measurement information is information indicating a situation in which Doppler measurement information is obtained in a reference transmission operation. The intermittent measurement information is information indicating a situation in which the Doppler measurement information is obtained in an intermittent transmission operation. The reference measurement information and the intermittent measurement information may include a Doppler shift frequency. In addition, the reference measurement information and the intermittent measurement information may include information indicating a high-pass filter characteristic of a wall filter according to the number of received Doppler signal values, and aliasing characteristic information regarding the Doppler shift frequency.

## Description

### BACKGROUND OF THE INVENTION

### 1. Field of the Invention

The present disclosure relates to an ultrasound diagnostic apparatus, a transmission pattern determination method, and a transmission pattern determination program, and particularly to a technique of intermittently transmitting ultrasound pulses.

### 2. Description of the Related Art

An ultrasound diagnostic apparatus that observes a tissue of a subject by transmitting and receiving ultrasound waves is widely used. Operation modes of the ultrasound diagnostic apparatus include a Doppler mode in which a blood flow velocity at an observation site is measured. The Doppler mode includes a color Doppler mode in which a color corresponding to the blood flow velocity is added to a B-mode image and displayed. In the measurement in the Doppler mode, ultrasound pulses are transmitted to the subject a plurality of times, and reflected waves a plurality of times generated from the subject are received. High-pass filter processing (wall filter processing) is performed on a plurality of received pulse signals based on the reflected waves received a plurality of times, and the blood flow velocity in a region of interest set on an ultrasound beam formed by the ultrasound pulses is calculated based on the plurality of received pulse signals after the high-pass filter processing.

As the high-pass filter, a filter such as a FIR filter or an IIR filter is used. The greater the number of the received pulse signals to be subjected to the high-pass filter processing is, the greater the number of tap coefficients of the high-pass filter can be. Therefore, the greater the number of the received pulse signals to be subjected to the high-pass filter processing is, the steeper the filter characteristics can be made. This allows for a greater attenuation amount in a low-frequency range while ensuring a high-pass frequency band. This reduces clutter in the displayed image.

In addition, in a case where a time interval for transmitting the ultrasound pulses is long, aliasing occurs in a narrow range of a Doppler shift frequency, and the measurable blood flow velocity range is narrowed. The shorter the time interval for transmitting the ultrasound pulses, the wider the measurable blood flow velocity range.

JP2010-17373A, JP2005-176997A, and JP2005-312632A disclose an ultrasound diagnostic apparatus that operates in a Doppler mode. These documents disclose a technique of thinning out any of a plurality of ultrasound pulses to be transmitted originally and intermittently transmitting the ultrasound pulses. In the intermittent transmission, a pattern of thinning out the ultrasound pulse in one raster direction is restricted by a pattern for thinning out the ultrasound pulse in another raster direction.

### SUMMARY OF THE INVENTION

In the Doppler mode operation, as the number of times of transmitting the ultrasound pulses increases, the number of received pulse signals increases, and the filter characteristics can be made steep to increase the effect of suppressing the clutter. However, as the number of times of transmitting the ultrasound pulses increases, a frame rate decreases. Here, the frame rate refers to the number of images generated per unit time.

Therefore, in order to increase the frame rate, it is considered to thin out any of a plurality of ultrasound pulses to be transmitted originally. However, in a case where such intermittent transmission is performed, aliasing may occur in a narrow range of the Doppler shift frequency, and the measurable blood flow velocity range may be narrowed.

An object of the present disclosure is to reduce the number of times of transmitting ultrasound pulses while maintaining Doppler measurement performance, such as a measurable blood flow velocity range and clutter suppression, for an ultrasound diagnostic apparatus that operates in a Doppler mode.

An ultrasound diagnostic apparatus according to the present disclosure comprises: a transmission unit that transmits a plurality of ultrasound pulses by using an ultrasound probe; a reception unit that receives, by using the ultrasound probe, reflected waves generated a plurality of times from a measurement object; and a processor configured to control the transmission unit and the reception unit, in which the processor is configured to generate a plurality of received Doppler signal values from a plurality of received pulse signals output from the reception unit in response to the reflected waves generated a plurality of times from the measurement object and to obtain Doppler measurement information based on each of the received Doppler signal values, and cause the transmission unit to execute intermittent transmission in which the ultrasound pulses are transmitted by omitting some of the ultrasound pulses based on an intermittent transmission pattern with respect to reference transmission in which the ultrasound pulses are transmitted a plurality of times at equal time intervals, and the intermittent transmission pattern is a transmission pattern that is selected from any of a plurality of different intermittent transmission pattern candidates based on error evaluation information in a case where a step including obtaining reference measurement information for the reference transmission, obtaining intermittent measurement information for the intermittent transmission, and obtaining the error evaluation information is executed for the plurality of different intermittent transmission pattern candidates, the reference measurement information indicating a situation in which the Doppler measurement information is obtained, the intermittent measurement information indicating a situation in which the Doppler measurement information is obtained, and the error evaluation information representing a difference between the reference measurement information and the intermittent measurement information.

In one embodiment, each of the reference measurement information and the intermittent measurement information includes a Doppler shift frequency, and the error evaluation information includes a value indicating a difference between the Doppler shift frequency included in the reference measurement information and the Doppler shift frequency included in the intermittent measurement information.

In one embodiment, the processor is configured to perform filter processing on the plurality of received Doppler signal values in accordance with the number of the received Doppler signal values, and generate the Doppler measurement information based on the plurality of received Doppler signal values on which the filter processing is performed, each of the reference measurement information and the intermittent measurement information includes information indicating a filter characteristic according to the number of the received Doppler signal values, and the error evaluation information includes a value indicating a difference between the filter characteristic included in the reference measurement information and the filter characteristic included in the intermittent measurement information.

In one embodiment, each of the reference measurement information and the intermittent measurement information includes aliasing characteristic information regarding a Doppler shift frequency, and the error evaluation information includes information indicating a difference between the aliasing characteristic information included in the reference measurement information and the aliasing characteristic information included in the intermittent measurement information.

In one embodiment, the processor is configured to generate the plurality of received Doppler signal values from a plurality of received pulse signals obtained in response to the intermittent transmission and to obtain the Doppler measurement information based on a plurality of interpolated received Doppler signal values obtained by performing interpolation processing on the plurality of received Doppler signal values to interpolate signal values omitted due to the intermittent transmission.

In one embodiment, the interpolation processing includes processing of interpolating the signal values omitted due to the intermittent transmission based on an interpolation function using an interpolation coefficient obtained in advance for the intermittent transmission pattern.

In one embodiment, the processor is configured to control the transmission unit and the reception unit to operate in a B-mode in a time period in which the transmission of the ultrasound pulses is thinned out during the intermittent transmission, and generate B-mode image data based on a signal output from the reception unit in the operation of the B-mode.

In one embodiment, the processor is configured to control the transmission unit and the reception unit so that a plurality of transmission and reception beams are formed, and cause the transmission unit to execute the intermittent transmission based on two complementary intermittent transmission patterns for two adjacent transmission and reception beams.

In addition, the present disclosure provides a transmission pattern determination method for obtaining an intermittent transmission pattern in an ultrasound diagnostic apparatus including a transmission unit that transmits a plurality of ultrasound pulses by using an ultrasound probe, a reception unit that receives, by using the ultrasound probe, reflected waves generated a plurality of times from a measurement object, and a processor configured to control the transmission unit and the reception unit, in which the processor is configured to generate a plurality of received Doppler signal values from a plurality of received pulse signals output from the reception unit in response to the reflected waves generated a plurality of times from the measurement object and to obtain Doppler measurement information based on each of the received Doppler signal values, and cause the transmission unit to execute intermittent transmission in which the ultrasound pulses are transmitted by omitting some of the ultrasound pulses based on the intermittent transmission pattern with respect to reference transmission in which the ultrasound pulses are transmitted a plurality of times at equal time intervals, the transmission pattern determination method comprising: executing, for a plurality of different intermittent transmission pattern candidates, a step including: obtaining reference measurement information for the reference transmission, the reference measurement information indicating a situation in which the Doppler measurement information is obtained; obtaining intermittent measurement information for the intermittent transmission, the intermittent measurement information indicating a situation in which the Doppler measurement information is obtained; and obtaining error evaluation information representing a difference between the reference measurement information and the intermittent measurement information; and selecting any of the plurality of different intermittent transmission pattern candidates as the intermittent transmission pattern, based on the error evaluation information.

In one embodiment, each of the reference measurement information and the intermittent measurement information includes a Doppler shift frequency, and the error evaluation information includes a value indicating a difference between the Doppler shift frequency included in the reference measurement information and the Doppler shift frequency included in the intermittent measurement information.

In one embodiment, the processor is configured to perform filter processing on the plurality of received Doppler signal values in accordance with the number of the received Doppler signal values, and generate the Doppler measurement information based on the plurality of received Doppler signal values on which the filter processing is performed, each of the reference measurement information and the intermittent measurement information includes information indicating a filter characteristic according to the number of the received Doppler signal values, and the error evaluation information includes a value indicating a difference between the filter characteristic included in the reference measurement information and the filter characteristic included in the intermittent measurement information.

In one embodiment, each of the reference measurement information and the intermittent measurement information includes aliasing characteristic information regarding a Doppler shift frequency, and the error evaluation information is a value indicating a difference between the aliasing characteristic information included in the reference measurement information and the aliasing characteristic information included in the intermittent measurement information.

In addition, the present disclosure provides a transmission pattern determination program for obtaining an intermittent transmission pattern in an ultrasound diagnostic apparatus including a transmission unit that transmits a plurality of ultrasound pulses by using an ultrasound probe, a reception unit that receives, by using the ultrasound probe, reflected waves generated a plurality of times from a measurement object, and a processor configured to control the transmission unit and the reception unit, in which the processor is configured to generate a plurality of received Doppler signal values from a plurality of received pulse signals output from the reception unit in response to the reflected waves generated a plurality of times from the measurement object and to obtain Doppler measurement information based on each of the received Doppler signal values, and cause the transmission unit to execute intermittent transmission in which the ultrasound pulses are transmitted by omitting some of the ultrasound pulses based on the intermittent transmission pattern with respect to reference transmission in which the ultrasound pulses are transmitted a plurality of times at equal time intervals, the transmission pattern determination program being read into a computer and causing the computer to execute a process comprising: executing, for a plurality of different intermittent transmission pattern candidates, a process including: obtaining reference measurement information for the reference transmission, the reference measurement information indicating a situation in which the Doppler measurement information is obtained; obtaining intermittent measurement information for the intermittent transmission, the intermittent measurement information indicating a situation in which the Doppler measurement information is obtained; and obtaining error evaluation information representing a difference between the reference measurement information and the intermittent measurement information; and selecting any of the plurality of different intermittent transmission pattern candidates as the intermittent transmission pattern, based on the error evaluation information.

According to the present disclosure, it is possible to reduce the number of times of transmitting ultrasound pulses while maintaining Doppler measurement performance for an ultrasound diagnostic apparatus that operates in a Doppler mode.

### BRIEF DESCRIPTION OF THE DRAWINGS

FIG. 1 is a diagram showing a configuration of an ultrasound diagnostic apparatus according to an embodiment of the present disclosure.
FIG. 2 is a diagram showing a reference transmission pattern and an intermittent transmission pattern.
FIG. 3 is a diagram illustrating interpolation processing for the intermittent transmission pattern.
FIG. 4 is a diagram showing examples of a reference transmission pattern and a plurality of intermittent transmission pattern candidates.
FIG. 5 is a diagram showing a transmittance coefficient characteristic of a wall filter.
FIG. 6 is a diagram showing Doppler shift frequencies for a reference transmission pattern and four intermittent transmission pattern candidates.
FIG. 7 is a diagram showing a Doppler shift frequency in a case where aliasing occurs.
FIG. 8 is a diagram showing a reference transmission pattern and an intermittent transmission pattern.
FIG. 9 is a diagram illustrating alternate execution of an operation of a B/Doppler hybrid mode and a B-mode operation in a time division manner.
FIG. 10 is a diagram showing an example in which two complementary intermittent transmission patterns are used for a pair of adjacent transmission and reception beams.

### DESCRIPTION OF THE PREFERRED EMBODIMENTS

### (1) Configuration and Basic Operation of Ultrasound Diagnostic Apparatus

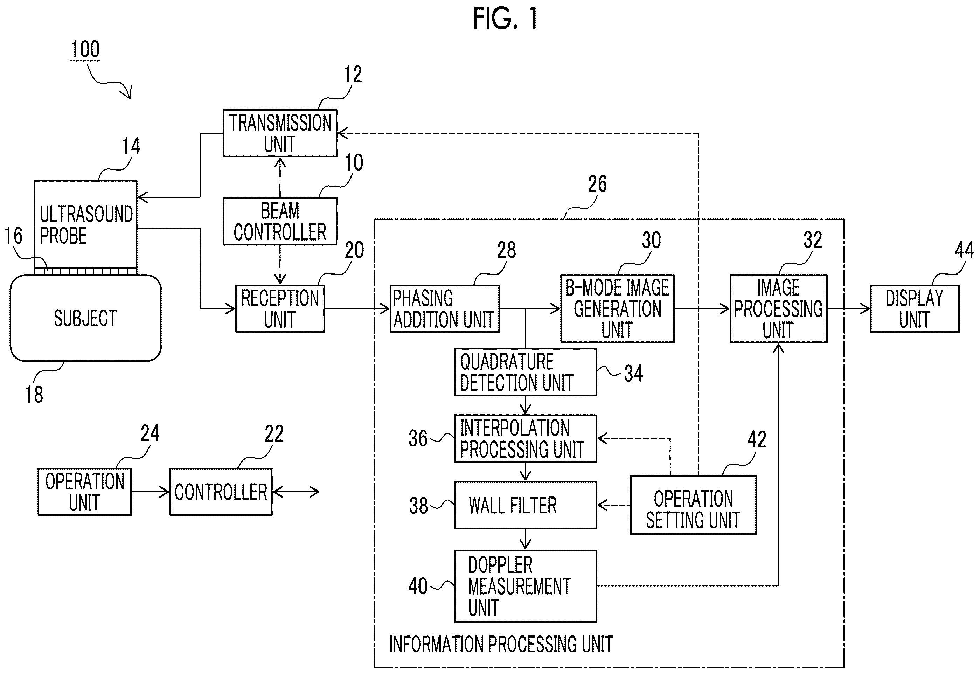

Each embodiment of the present disclosure will be described with reference to the drawings. The same components shown in a plurality of drawings are denoted by the same reference numerals, and the description thereof will not be repeated. FIG. 1 shows a configuration of an ultrasound diagnostic apparatus 100 according to an embodiment of the present disclosure. The ultrasound diagnostic apparatus 100 comprises a beam controller 10, a transmission unit 12, an ultrasound probe 14, a reception unit 20, a controller 22, an operation unit 24, an information processing unit 26, and a display unit 44. The reception unit 20 and the transmission unit 12 may include an electric circuit as a reception circuit and an electric circuit as a transmission circuit, respectively. In addition, the reception unit 20 and the transmission unit 12 may be hardware including an electric circuit and a processor that controls the electric circuit.

The information processing unit 26 comprises a phasing addition unit 28, a B-mode image generation unit 30, an image processing unit 32, a quadrature detection unit 34, an interpolation processing unit 36, a wall filter 38, a Doppler measurement unit 40, and an operation setting unit 42. The information processing unit 26 may be configured by one or a plurality of processors that execute a program to realize functions of these components (the phasing addition unit 28, the B-mode image generation unit 30, the image processing unit 32, the quadrature detection unit 34, the interpolation processing unit 36, the wall filter 38, the Doppler measurement unit 40, and the operation setting unit 42). The controller 22 performs overall control of the ultrasound diagnostic apparatus 100. The operation unit 24 includes a keyboard, a mouse, a lever, a button, a voice recognition device, and the like, and outputs information regarding a user's operation to the controller 22. The controller 22 controls the ultrasound diagnostic apparatus 100 in response to an operation on the operation unit 24.

The operation unit 24 may constitute a user interface for operating the ultrasound diagnostic apparatus 100 together with the display unit 44. For example, the operation unit 24 may include a touch panel on a display screen of the display unit 44. In addition, the operation unit 24 may have a function of operating a keyboard, a lever, a button, or the like that is virtually configured on the display screen.

An outline of an operation of the ultrasound diagnostic apparatus 100 will be described. The ultrasound diagnostic apparatus 100 transmits ultrasound waves from the ultrasound probe 14 to a subject 18 as a measurement object, and receives ultrasound waves reflected in the subject 18, that is, reflected waves generated in the subject 18 by using the ultrasound probe 14. The ultrasound diagnostic apparatus 100 executes measurements in a B-mode and a Doppler mode.

In the operation of the B-mode, the ultrasound diagnostic apparatus 100 generates B-mode image data based on the reflected waves received from the subject 18 and displays a B-mode image on the display unit 44. In the operation of the Doppler mode, the ultrasound diagnostic apparatus 100 generates B-mode color Doppler image data that indicates an image in which a color corresponding to a blood flow velocity is applied to a B-mode image, and displays the B-mode color Doppler image on the display unit 44.

A specific configuration of the ultrasound diagnostic apparatus 100 and specific processing executed by the ultrasound diagnostic apparatus 100 will be described. The ultrasound probe 14 is in a state of being in contact with a surface of the subject 18. The ultrasound probe 14 comprises a plurality of transducer elements 16. The transmission unit 12 outputs a transmission signal to each of the transducer elements 16 of the ultrasound probe 14 under the control of the beam controller 10. As a result, the ultrasound waves are transmitted from the ultrasound probe 14. The beam controller 10 may control the transmission unit 12 to form a transmission beam in the ultrasound probe 14 and scan the subject 18 with the transmission beam. That is, the transmission unit 12 may adjust a delay time or a level of each transmission signal in accordance with the control of the beam controller 10, form a transmission beam in the ultrasound probe 14, and scan the subject 18 with the transmission beam. In addition, the transmission unit 12 may adjust the delay time or the level of each transmission signal in accordance with the control of the beam controller 10, and form a plurality of transmission beams in the ultrasound probe 14.

In a case where the reflected wave generated in the subject 18 is received by each transducer element 16 of the ultrasound probe 14, each transducer element 16 outputs a reception signal corresponding to the received ultrasound wave to the reception unit 20. The reception unit 20 performs processing, such as amplification and frequency band limitation, on the reception signal output from each transducer element 16 in accordance with the control of the beam controller 10, and outputs the processed reception signal to the information processing unit 26. The information processing unit 26 performs processing executed by each component (the phasing addition unit 28, the B-mode image generation unit 30, the image processing unit 32, the quadrature detection unit 34, the interpolation processing unit 36, the wall filter 38, and the Doppler measurement unit 40) on each reception signal.

The phasing addition unit 28 performs phasing addition of a plurality of the reception signals output from the reception unit 20 for the plurality of transducer elements 16 to generate a phase-adjusted reception signal. As a result, a phase-adjusted reception signal is generated through phase adjustment and addition such that the reception signals based on the ultrasound waves received in a specific direction strengthen each other, and a reception beam is formed in the specific direction. The phasing addition unit 28 outputs the phase-adjusted reception signal to the B-mode image generation unit 30 during the B mode operation. In addition, the phasing addition unit 28 outputs the phase-adjusted reception signal to the quadrature detection unit 34 during the operation in the Doppler mode.

The operation in the B-mode will be described. The phasing addition unit 28 generates each phase-adjusted reception signal based on the ultrasonic wave received in each direction of the reception beam scanned in the subject 18, and outputs the phase-adjusted reception signal to the B-mode image generation unit 30 as a B-mode image reception signal. The B-mode image generation unit 30 generates B-mode image data based on the B-mode image reception signal obtained in each reception beam direction, and outputs the B-mode image data to the image processing unit 32. The B-mode image data based on one scan of the transmission beam and the reception beam (hereinafter, referred to as transmission and reception beams) is image data for one frame and corresponds to one B-mode image.

The beam controller 10, the transmission unit 12, the ultrasound probe 14, the reception unit 20, the phasing addition unit 28, and the B-mode image generation unit 30 generate the B-mode image data one after another in association with repetitive scan of the transmission and reception beams, and output each B-mode image data to the image processing unit 32. In a case where a plurality of transmission and reception beams are formed in the ultrasound probe 14, the beam controller 10, the transmission unit 12, the ultrasound probe 14, the reception unit 20, the phasing addition unit 28, and the B-mode image generation unit 30 sequentially generate the B-mode image data based on the B-mode image reception signal obtained for each of the plurality of transmission and reception beams, and output the B-mode image data to the image processing unit 32. The image processing unit 32 generates a video signal for displaying the B-mode image based on the B-mode image data, and outputs the video signal to the display unit 44. The display unit 44 displays the B-mode image based on the video signal.

### (2) Doppler Mode

### (2-1) Outline of Operation

The operation in the Doppler mode will be described. The Doppler mode operation according to the present embodiment includes a reference operation and an intermittent transmission operation. In the reference operation, reference transmission in which ultrasound pulses are transmitted n times at equal time intervals for one transmission and reception beam is performed. As a result, reflected waves are received n times, and phase-adjusted reception signals are generated n times. Here, n is an integer of 2 or more. The Doppler measurement unit 40 generates Doppler measurement information based on n received Doppler signal values obtained by performing quadrature detection of the phase-adjusted reception signals generated n times.

The Doppler measurement information may include color Doppler data. The color Doppler data indicates blood flow velocity using color on a B-mode image of a region where scanning with the transmission and reception beams is performed. The color Doppler data may be data in which, for example, a blue color is applied to a region where the blood flows in a direction away from the ultrasound probe 14, a red color is applied to a region where the blood flows in a direction approaching the ultrasound probe 14, and brightness is increased in regions where the blood flow velocity is greater. The Doppler measurement information generated by the Doppler measurement unit 40 may include various types of information related to the blood flow velocity in the region where scanning with the transmission and reception beams is performed, in addition to the color Doppler data.

In the intermittent transmission operation, intermittent transmission in which the ultrasound pulses are transmitted m times, which are less than n times, at time intervals according to an intermittent transmission pattern for one transmission and reception beam is performed. As a result, reflected waves are received m times, and phase-adjusted reception signals are generated m times. Here, the intermittent transmission pattern indicates predetermined time intervals that are not equal time intervals. That is, in the intermittent transmission operation, any of the n ultrasound pulses transmitted at equal time intervals in the reference operation is thinned out in accordance with the intermittent transmission pattern. The Doppler measurement unit 40 generates Doppler measurement information based on m received Doppler signal values obtained by performing quadrature detection of the phase-adjusted reception signals generated m times. The m received Doppler signal values are subjected to interpolation processing, and the received Doppler signal value omitted with respect to the reference operation is interpolated.

The intermittent transmission pattern is obtained by a transmission pattern determination method described below. In the transmission pattern determination method, in a case where the intermittent transmission operation is executed according to each of a plurality of intermittent transmission pattern candidates, an error evaluation value representing a difference between a situation of the reference operation and a situation of the intermittent transmission operation is obtained for each of the plurality of intermittent transmission pattern candidates. Then, among the plurality of intermittent transmission pattern candidates, an intermittent transmission pattern candidate having a minimum error evaluation value or an intermittent transmission pattern candidate having an error evaluation value equal to or less than a predetermined threshold value is selected as the intermittent transmission pattern used in the intermittent transmission operation. In the transmission pattern determination method, an interpolation coefficient required for the processing of interpolating the received Doppler signal value is further determined for the determined intermittent transmission pattern.

### (2-2) Reference Operation

The reference operation will be specifically described. In the reference operation, the ultrasound pulses are transmitted n times for one transmission and reception beam. n received pulse signals are output as n phase-adjusted reception signals to the quadrature detection unit 34 from the phasing addition unit 28. The quadrature detection unit 34 generates n received Doppler signal values by performing quadrature detection on the n received pulse signals with a local signal having a frequency of the transmitted ultrasound pulse.

In the reference operation, the interpolation processing unit 36 does not execute the interpolation processing. The quadrature detection unit 34 outputs the n received Doppler signal values to the wall filter 38. The wall filter 38 performs high-pass filter processing on the n received Doppler signal values, and outputs the n received Doppler signal values after the high-pass filter processing to the Doppler measurement unit 40.

The Doppler measurement unit 40 generates color Doppler data as one piece of information included in the Doppler measurement information, based on the n received Doppler signal values acquired for each of a plurality of transmission and reception beams having different positions or directions.

The Doppler measurement unit 40 obtains a blood flow velocity v(r) in a depth direction of the transmission and reception beam based on the n received Doppler signal values, for example, in accordance with autocorrelation processing disclosed in JP2016-087302A. Here, v(r) indicates a blood flow velocity at a position of a depth r. The Doppler measurement unit 40 generates color Doppler data based on the blood flow velocity v(r) in the depth direction at each depth r obtained for each of the plurality of transmission and reception beams having different positions or directions, and outputs the color Doppler data to the image processing unit 32. Hereinafter, specific processing of obtaining the blood flow velocity v(r) will be described.

A received Doppler signal value acquired at the q-th (q = 1 to n) position among the first to n-th received Doppler signal values is represented by the following (Equation 1).
$z \left(r, q\right) = I \left(r, q\right) + j ⋅ Q \left(r, q\right)$

r is a coordinate value in the depth direction. I(r,q) indicates an in-phase component of the received Doppler signal value, and Q(r,q) indicates a quadrature component of the received Doppler signal value. j is an imaginary unit. The Doppler measurement unit 40 obtains an autocorrelation value A(r) for n received Doppler signal values z(r,1), z(r,2), ..., z(r,n) in accordance with (Equation 2).
$A \left(r\right) = {\sum }_{q = 1}^{n - 1} z \left(r, q\right) ⋅ z {\left(r,q+1\right)}^{∗}$

Here, the superscript "*" represents a complex conjugate. The Doppler measurement unit 40 obtains the blood flow velocity v(r) in accordance with (Equation 3) by using a real part Re[A(r)] and an imaginary part Im[A(r)] of the autocorrelation value A(r).
$v \left(r\right) = \frac{c}{2 {f}_{0}} ⋅ \frac{1}{2 πT} arctan \left[\frac{Im \left[A\left(r\right)\right]}{Im \left[A\left(r\right)\right]}\right]$

c is a propagation velocity of the ultrasound pulse propagating through the subject 18. f₀ is a frequency of the ultrasound pulse transmitted from the ultrasound probe 14. T is a time interval for transmitting n ultrasound pulses. A portion on the right side of (Equation 3) to the right of c/(2f₀) represents a Doppler shift frequency fd(r). The blood flow velocity v(r) represents a component of the blood flow velocity in the depth direction at the depth r. In a case where an angle φ formed by the blood flow direction with respect to the depth direction is known, v(r)/cosφ is the blood flow velocity.

(Equation 2) and (Equation 3) mean that a difference in vector phase angle between the received Doppler signal values adjacent to each other on a time axis is averaged for the n received Doppler signal values, and the Doppler shift frequency or the blood flow velocity is obtained based on the time interval T and the averaged difference in vector phase angle.

In a case where it is not necessary to obtain an absolute value of the blood flow velocity (for example, a value in the MKSA unit system) such as in a case of measuring a blood flow velocity distribution, the blood flow velocity may be a value obtained by multiplying the value obtained in (Equation 3) by an arbitrary constant. In addition, instead of the blood flow velocity, a Doppler shift frequency, which is a portion on the right side of (Equation 3) to the right of c/(2f₀), may be used.

The image processing unit 32 generates data indicating a B-mode color Doppler image in which a color corresponding to the blood flow velocity is applied to the B-mode image, based on the B-mode image data and the color Doppler data. The image processing unit 32 generates a video signal based on the B-mode color Doppler image data, and outputs the video signal to the display unit 44. The display unit 44 displays the B-mode color Doppler image based on the video signal.

The Doppler measurement unit 40 may use the blood flow velocity v(r) or the Doppler shift frequency fd(r) at each depth r obtained for each of the plurality of transmission and reception beams having different positions or directions as one piece of the Doppler measurement information.

As described above, the Doppler measurement information generated by the Doppler measurement unit 40 includes, in addition to the color Doppler data, the blood flow velocity v(r) or the Doppler shift frequency fd(r) obtained for each of the transmission and reception beams having different positions or directions.

In the reference operation, the Doppler measurement unit 40 generates Doppler measurement information based on the n received Doppler signal values on which the high-pass filter processing is performed by the wall filter 38. The received Doppler signal value output from the quadrature detection unit 34 includes low-frequency noise based on the movement of the tissue in the subject 18, and is a cause of unnecessary components contained in the value of the Doppler shift frequency. For example, this causes noise referred to as clutter in the color Doppler data. Therefore, the wall filter 38 suppresses unnecessary components contained in the value of the Doppler shift frequency.

### (2-3) Intermittent Transmission Operation

The intermittent transmission operation will be specifically described. In the intermittent transmission operation, for one transmission and reception beam, the ultrasound pulses are transmitted at time intervals according to the intermittent transmission pattern, for a smaller number of times than in the reference operation. The operation setting unit 42 stores intermittent transmission pattern data in advance.

The intermittent transmission pattern data defines a transmission timing in accordance with the intermittent transmission pattern. The intermittent transmission pattern is determined such that a range of measurable frequencies is not narrowed due to aliasing of the Doppler shift frequency, or the accuracy of the Doppler shift frequency (blood flow velocity) is maintained. The operation setting unit 42 controls the transmission unit 12 based on the intermittent transmission pattern data, and causes the transmission unit 12 to execute the intermittent transmission operation according to the intermittent transmission pattern.

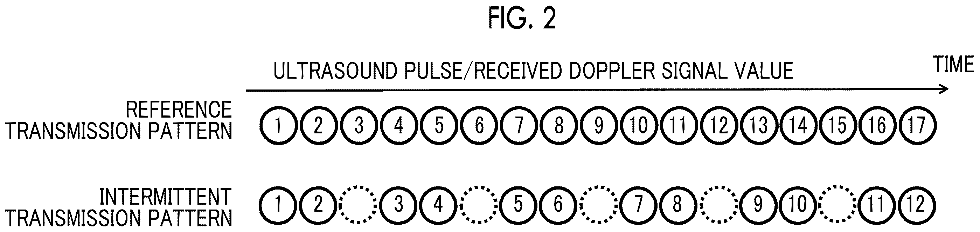

FIG. 2 conceptually shows a reference transmission pattern in the reference operation and an intermittent transmission pattern in the intermittent transmission operation. A horizontal axis indicates time. The circular mark indicated by the solid line indicates a timing at which the ultrasound pulse is transmitted. The circular mark indicated by the broken line indicates an omission timing at which the transmission of the ultrasound pulses is thinned out in the intermittent transmission operation. The numbers 1 to 17 for specifying the transmission timings are assigned to the respective transmission timings.

In the example shown in FIG. 2, in the reference operation according to the reference transmission pattern, the ultrasound pulses are transmitted n = 17 times for one transmission and reception beam. In the intermittent transmission operation according to the intermittent transmission pattern, the timings indicated by the transmission pulse numbers 3, 6, 9, 12, and 15 in the reference transmission pattern are the omission timings. The number of times the ultrasound pulses are transmitted is m = 12.

In the intermittent transmission operation, the ultrasound pulses are transmitted m times for one transmission and reception beam. The phasing addition unit 28 generates the phase-adjusted reception signal as the received pulse signal m times, and outputs the phase-adjusted reception signal to the quadrature detection unit 34. The quadrature detection unit 34 performs quadrature detection on the m received pulse signals to generate m received Doppler signal values, and outputs the m received Doppler signal values to the interpolation processing unit 36.

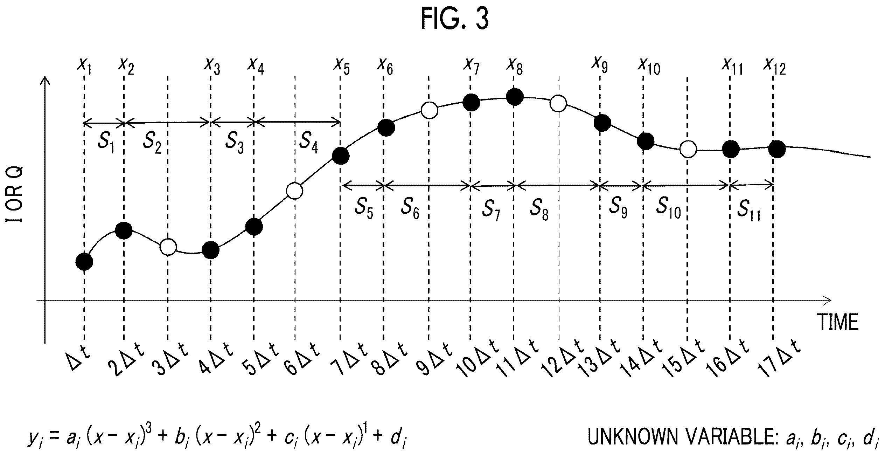

The interpolation processing executed by the interpolation processing unit 36 will be described. FIG. 3 is a diagram illustrating the interpolation processing for the intermittent transmission pattern. A horizontal axis indicates time, and a vertical axis indicates an in-phase component I or a quadrature component Q (hereinafter, may be referred to as a signal value) of the received Doppler signal value. The time interval of the reference transmission pattern is Δt. In the example shown in FIG. 3, with respect to the reference transmission pattern, signal values at times x = 3Δt, 6Δt, 9Δt, 12Δt, and 15Δt are omitted. The interpolation processing is executed on each of the in-phase component I and the quadrature component Q of the received Doppler signal value.

The interpolation processing unit 36 obtains interpolation values at times x = 3Δt, 6Δt, 9Δt, 12Δt, and 15Δt based on the m = 12 received Doppler signal values (a set of the in-phase component I or the quadrature component Q is regarded as one received Doppler signal value). The interpolation values are obtained by obtaining an interpolation function using times x = Δt, 2Δt, 4Δt, 5Δt, 7Δt, 8Δt, 10Δt, 11Δt, 13Δt, 14Δt, 16Δt, and 17Δt and signal values y₁ to y₁₂ at each of these times. Note that, in FIG. 3, times x = Δt, 2Δt, 4Δt, 5Δt, 7Δt, 8Δt, 10Δt, 11Δt, 13Δt, 14Δt, 16Δt, and 17Δt are represented by x₁ to x₁₂, respectively. The interpolation function indicates a straight line or a curve passing through an actual measurement point corresponding to each signal value on a time-signal value plane.

Such interpolation processing includes spline interpolation processing. For example, in cubic spline interpolation processing, coefficients of a cubic interpolation function representing a value between an actual measurement point corresponding to time xⱼ and an actual measurement point corresponding to time xⱼ₊₁ are obtained. This interpolation function is represented by the following (Equation 4). Here, j is any integer of 1 to 11. ${S}_{j} \left(x\right) = {a}_{j} {\left(x-{x}_{j}\right)}^{3} + {b}_{j} {\left(x-{x}_{j}\right)}^{2} + {c}_{j} {\left(x-{x}_{j}\right)}^{1} + {d}_{j}$

Interpolation coefficients aⱼ, bⱼ, cⱼ, and dⱼ are obtained by using the signal values y₁ to y₁₂ at times x = x₁ to x₁₂. In the example shown in FIG. 3, sets of the interpolation coefficients aⱼ, bⱼ, cⱼ, and dⱼ are obtained for j = 1 to 11, and 44 interpolation coefficients are obtained. The interpolation value between the actual measurement point corresponding to time xⱼ and the actual measurement point corresponding to time xⱼ₊₁ is obtained by providing x satisfying xⱼ < x < xⱼ₊₁ to the right side of (Equation 4).

Here, in a case where the intermittent transmission pattern is predetermined, the interpolation coefficients aⱼ, bⱼ, cⱼ, and dⱼ are also predetermined. Therefore, the operation setting unit 42 according to the present embodiment stores the interpolation coefficients aⱼ, bⱼ, cⱼ, and dⱼ corresponding to the intermittent transmission pattern data together with the intermittent transmission pattern data used in the intermittent operation.

The operation setting unit 42 outputs the interpolation coefficients corresponding to the intermittent transmission pattern to the interpolation processing unit 36. The interpolation processing unit 36 uses the interpolation coefficients output from the operation setting unit 42 to execute interpolation processing on m received Doppler signal values based on (Equation 4), and generates n interpolated received Doppler signal values.

Here, the cubic spline interpolation processing is taken as the interpolation processing, but the order of the spline interpolation processing is arbitrary. In addition, other interpolation processing, such as Lagrange interpolation processing and processing based on a least-squares method, may be used. According to general interpolation processing, in a case where the intermittent transmission pattern is predetermined, the interpolation coefficients are also predetermined. The operation setting unit 42 stores the interpolation coefficients corresponding to the intermittent transmission pattern data together with the intermittent transmission pattern data used in the intermittent operation. The interpolation processing unit 36 executes interpolation processing on m received Doppler signal values using the interpolation coefficients stored in advance, and generates n interpolated received Doppler signal values.

The wall filter 38 performs high-pass filter processing on the n interpolated received Doppler signal values, and outputs the n interpolated received Doppler signal values after the high-pass filter processing to the Doppler measurement unit 40. The Doppler measurement unit 40 generates Doppler measurement information based on the n interpolated received Doppler signal values acquired for each of a plurality of transmission and reception beams having different positions or directions.

With such an intermittent transmission operation, the time required to acquire the Doppler measurement information is shorter than that in the reference operation. As a result, the frame rate can be improved as compared with the reference operation. In addition, by using an intermittent transmission pattern determined by the transmission pattern determination method described below, the phenomenon in which the range of the measurable Doppler shift frequency is narrowed due to aliasing of the Doppler shift frequency is suppressed, or the accuracy of the Doppler shift frequency (blood flow velocity) is maintained.

### (3) Transmission Pattern Determination Method

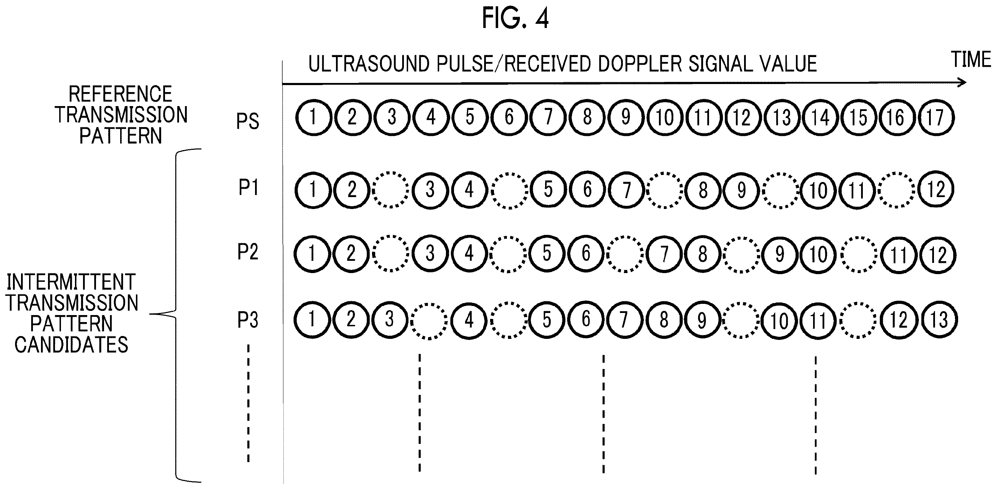

Next, the transmission pattern determination method will be described. In the transmission pattern determination method, from among a plurality of intermittent transmission pattern candidates (hereinafter, referred to as intermittent transmission pattern candidates), one that satisfies a predetermined condition is selected as the intermittent transmission pattern. FIG. 4 conceptually shows examples of a reference transmission pattern in the reference operation and a plurality of intermittent transmission pattern candidates. Definitions of the reference numerals, figures, and the like shown in the drawing are the same as those in FIG. 2.

In the example shown in FIG. 4, in reference transmission PS according to the reference transmission pattern, the ultrasound pulses are transmitted n = 17 times for one transmission and reception beam. In the intermittent transmission operation according to an intermittent transmission pattern candidate P1 shown at the top, the timings indicated by the transmission pulse numbers 3, 6, 10, 13, and 16 in the reference transmission pattern are the omission timings. The number of times the ultrasound pulses are transmitted is m = 12. In the intermittent transmission operation according to an intermittent transmission pattern candidate P2 shown as the second from the top, the timings indicated by the transmission pulse numbers 3, 6, 9, 12, and 15 in the reference transmission pattern are the omission timings. The number of times the ultrasound pulses are transmitted is m = 12. In the intermittent transmission operation according to an intermittent transmission pattern candidate P3 shown as the third from the top, the timings indicated by the transmission pulse numbers 4, 6, 12, and 15 in the reference transmission pattern are the omission timings. The number of times the ultrasound pulses are transmitted is m = 13.

In the transmission pattern determination method, as a first process, a transmittance coefficient characteristic of the wall filter 38 is obtained for the reference transmission pattern and each of the plurality of intermittent transmission pattern candidates. Here, the transmittance coefficient characteristic is a characteristic in which the transmittance coefficient is associated with the Doppler shift frequency. In addition, the wall filter 38 is a digital filter, such as an FIR filter or an IIR filter, whose transmittance coefficient characteristics are determined by given filter tap coefficients.

The transmittance coefficient characteristics of the wall filter 38 are different depending on a position on a time axis at which the received Doppler signal values are multiplied by the filter tap coefficients, or on the number of the filter tap coefficients. The received Doppler signal values according to the reference transmission pattern and each of the plurality of intermittent transmission pattern candidates have different arrangements on the time axis. Therefore, the transmittance coefficient characteristics of the wall filter 38 corresponding to the reference transmission pattern and each of the plurality of intermittent transmission pattern candidates are also different.

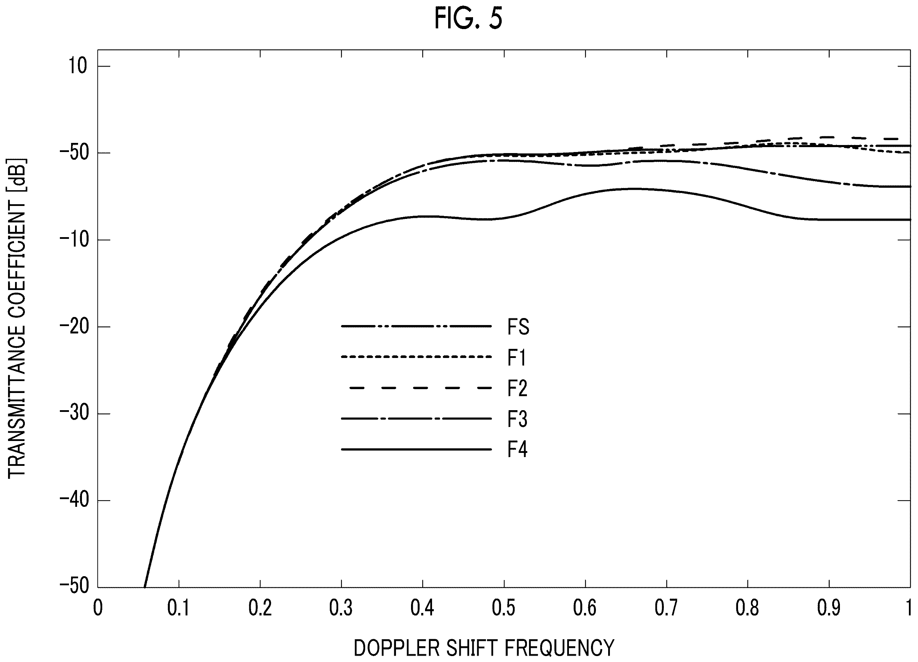

Hereinafter, a second process of selecting any one of four intermittent transmission pattern candidates as the intermittent transmission pattern will be described. FIG. 5 shows a transmittance coefficient characteristic FS of the wall filter 38 for the reference transmission pattern. In addition, transmittance coefficient characteristics F1 to F4 of the wall filter 38 for the four intermittent transmission pattern candidates are shown. In the second process, an error evaluation value efᵢ (i = 1 to 4) indicating a difference from the transmittance coefficient characteristic FS is obtained for each of the transmittance coefficient characteristics F1 to F4.

For example, the error evaluation value efᵢ may be defined as an average value of a value obtained by subtracting a true numerical value of the transmittance coefficient FS from a true numerical value of a transmittance coefficient Fi. In addition, the error evaluation value efᵢ may also be defined as an average value of a value obtained by squaring the value obtained by subtracting the true numerical value of the transmittance coefficient FS from the true numerical value of the transmittance coefficient Fi. These average values may be defined as average values in a frequency range lower than a 3 dB cut-off frequency. In addition, the error evaluation value efᵢ may be obtained based on a decibel value instead of the true numerical value.

In a third process, among the four intermittent transmission pattern candidates, one having the smallest error evaluation value efᵢ or one having an error evaluation value efᵢ equal to or less than a predetermined threshold value is selected as the intermittent transmission pattern.

In the transmission pattern determination method, from among the plurality of intermittent transmission pattern candidates, one that is to be the intermittent transmission pattern may be selected based on the difference in the Doppler shift frequency. In this case, as a first process, for a certain transmission and reception beam and a certain depth r, the Doppler shift frequency is obtained for the reference transmission pattern and each of the plurality of intermittent transmission pattern candidates. For example, the Doppler shift frequency with respect to the ideal value of the Doppler shift frequency may be obtained by actual measurement or simulation.

The Doppler shift frequency is obtained by averaging the difference in vector phase angle between the received Doppler signal values adjacent to each other on the time axis for the m received Doppler signal values, and by using the time interval T of the ultrasound pulses in the reference operation and the averaged difference in vector phase angle.

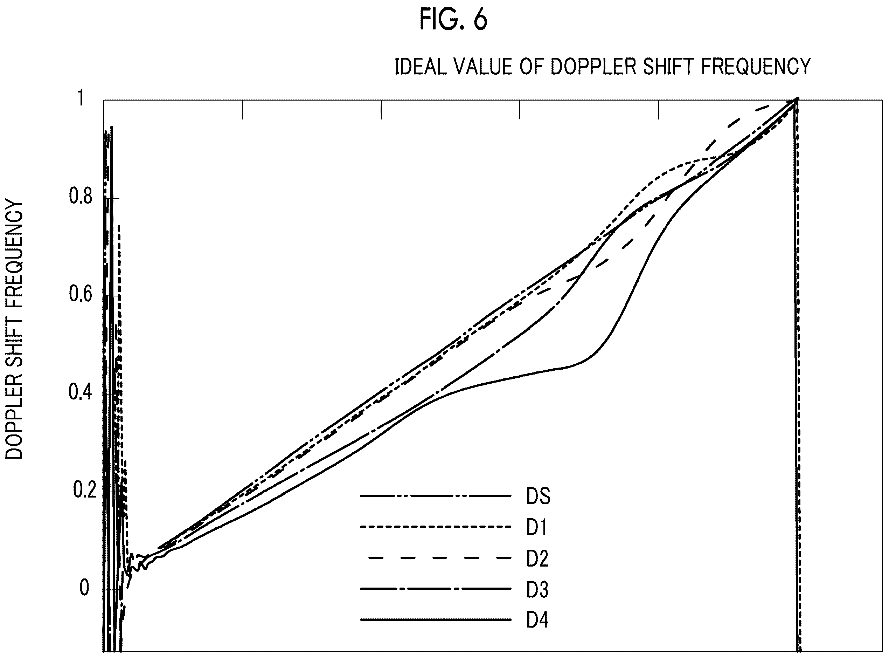

FIG. 6 shows a Doppler shift frequency DS for the reference transmission pattern. In addition, Doppler shift frequencies D1 to D4 for the four intermittent transmission pattern candidates are shown. A horizontal axis indicates the ideal value of the Doppler shift frequency, and a vertical axis indicates the Doppler shift frequency. In a case where the ideal value of the Doppler shift frequency is denoted by fd0, the Doppler shift frequency DS for the reference transmission pattern is DS = fd0.

In a second process, an error evaluation value edᵢ (i = 1 to 4) indicating a difference from the Doppler shift frequency DS is obtained for each of the Doppler shift frequencies D1 to D4. For example, the error evaluation value edi may be defined as an average value of a value obtained by subtracting a true numerical value of the Doppler shift frequency DS from a true numerical value of the Doppler shift frequency Di. In addition, the error evaluation value edᵢ may be defined as an average value of a value obtained by squaring the value obtained by subtracting the true numerical value of the Doppler shift frequency DS from the true numerical value of the Doppler shift frequency Di. These average values may be defined as average values in a predetermined range of the ideal value of the predetermined Doppler shift frequency.

In a third process, among the four intermittent transmission pattern candidates, one having the smallest error evaluation value edᵢ or one having an error evaluation value edᵢ equal to or less than a predetermined threshold value is selected as the intermittent transmission pattern.

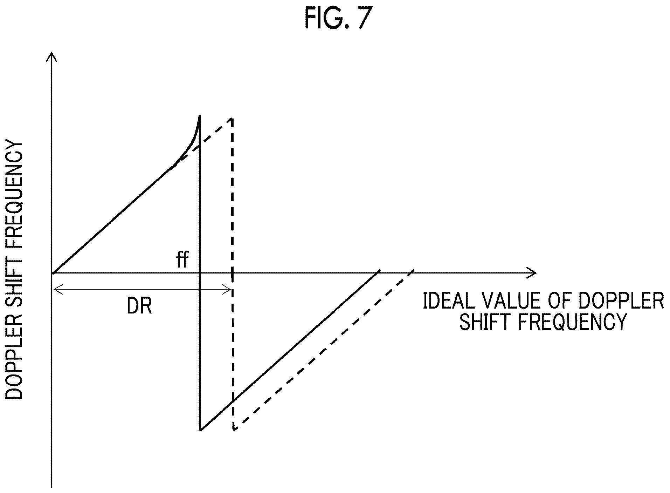

In the transmission pattern determination method, the intermittent transmission pattern may be selected from the intermittent transmission pattern candidates under a condition in which aliasing does not occur in the Doppler shift frequency within a predetermined range of the ideal value of the Doppler shift frequency. Here, aliasing refers to a phenomenon in which, in a case where the value of the Doppler shift frequency changes, the polarity changes discontinuously and the value changes discontinuously from the maximum value to the minimum value or from the minimum value to the maximum value. FIG. 7 shows a Doppler shift frequency in a case where aliasing occurs. The broken line indicates an ideal Doppler shift frequency, and the solid line indicates a Doppler shift frequency obtained for the intermittent transmission pattern candidate. In the example shown in FIG. 7, aliasing occurs at a frequency ff within a predetermined range DR of the ideal value of the Doppler shift frequency. The intermittent transmission pattern candidate from which this Doppler shift frequency is obtained is not selected as the intermittent transmission pattern.

In addition, a ratio of a Doppler shift frequency bandwidth in which no aliasing occurs with respect to the intermittent transmission pattern to a Doppler shift frequency bandwidth in which no aliasing occurs with respect to the reference transmission pattern may be obtained as an error evaluation value egᵢ. In this case, among the four intermittent transmission pattern candidates, one having the smallest error evaluation value egᵢ or one having an error evaluation value egᵢ equal to or less than a predetermined threshold value is selected as the intermittent transmission pattern.

In the above, an example has been described in which any of the plurality of intermittent transmission pattern candidates is selected as the intermittent transmission pattern based on the error evaluation values efᵢ, edᵢ, or egᵢ. The intermittent transmission pattern may be selected based on a weighted average value of at least two of efᵢ, edᵢ, and egᵢ. The weighted average value is defined as, for example, e = (Wf·efᵢ + Wd·edᵢ + Wg·egᵢ)/(Wf + Wd + Wg). Each of Wf, Wd, and Wg is a number of 0 or more and 1 or less, and Wf + Wd + Wg is a non-zero value.

The above describes a method for determining an intermittent transmission pattern from the four intermittent transmission pattern candidates. The intermittent transmission pattern determination method may be for two, three, or five or more intermittent transmission pattern candidates in addition to the four intermittent transmission pattern candidates.

The intermittent transmission pattern determination method will be summarized. The intermittent transmission pattern determination method includes obtaining reference measurement information for reference transmission and intermittent measurement information for intermittent transmission, and obtaining error evaluation information representing a difference between the reference measurement information and the intermittent measurement information. Here, the reference measurement information is information indicating a situation in which Doppler measurement information is obtained in a reference transmission operation. The intermittent measurement information is information indicating a situation in which the Doppler measurement information is obtained in an intermittent transmission operation. The reference measurement information and the intermittent measurement information may include a Doppler shift frequency. In addition, the reference measurement information and the intermittent measurement information may include information indicating a high-pass filter characteristic of the wall filter 38 according to the number of received Doppler signal values. In addition, the reference measurement information and the intermittent measurement information may include aliasing characteristic information for the Doppler shift frequency. The aliasing characteristic information may be information indicating that aliasing occurs in a frequency band in which no aliasing occurs in an ideal operation.

The error evaluation information may include a value indicating a difference between the Doppler shift frequency included in the reference measurement information and the Doppler shift frequency included in the intermittent measurement information, for example, the error evaluation value edᵢ. In addition, the error evaluation information may include a value indicating a difference between the high-pass filter characteristic (filter characteristic) included in the reference measurement information and the high-pass filter characteristic (filter characteristic) included in the intermittent measurement information, for example, the error evaluation value efᵢ. In addition, the error evaluation information may include information indicating that aliasing occurs within a predetermined range DR of the ideal value of the Doppler shift frequency as information indicating a difference between the aliasing characteristic information included in the reference measurement information and the aliasing characteristic information included in the intermittent measurement information. In addition, the error evaluation information may include the error evaluation value egᵢ as the information indicating the difference between the aliasing characteristic information included in the reference measurement information and the aliasing characteristic information included in the intermittent measurement information.

The transmission pattern determination method may be executed by a computer provided separately from the ultrasound diagnostic apparatus 100. The computer executes a program (transmission pattern determination program) for executing each process of the transmission pattern determination method. The computer that has determined the intermittent transmission pattern obtains interpolation coefficients corresponding to the intermittent transmission pattern data, and stores the interpolation coefficients in the operation setting unit 42 together with the intermittent transmission pattern data. The transmission pattern determination method may also be performed by actual measurement using the ultrasound diagnostic apparatus 100. In this case, the information processing unit 26 may execute the transmission pattern determination program and execute each processing in the transmission pattern determination method, instead of the computer provided separately from the ultrasound diagnostic apparatus 100.

According to the transmission pattern determination method, the intermittent transmission pattern is determined such that the time required to acquire the Doppler measurement information is shorter than that in the reference operation while maintaining the accuracy of the Doppler shift frequency (blood flow velocity) and suppressing the phenomenon in which the range of the measurable frequency is narrowed due to aliasing of the Doppler shift frequency. In addition, the intermittent pattern is determined such that the characteristics of the wall filter 38 are close to the characteristics in the reference operation, and the effect of suppressing the artifact such as clutter is maintained.

### (4) Application Operation

### (4-1) B/Doppler Hybrid Mode

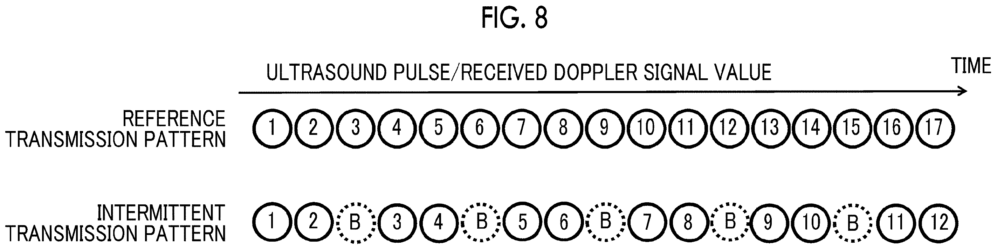

In a time period during which the ultrasound pulses are thinned out in the intermittent transmission operation, the ultrasound pulses in the B-mode operation may be transmitted. FIG. 8 conceptually shows a reference transmission pattern in the reference operation and an intermittent transmission pattern in the intermittent transmission operation. In the example shown in FIG. 8, in the reference operation according to the reference transmission pattern, the ultrasound pulses are transmitted n = 17 times for one transmission and reception beam. In the intermittent transmission operation according to the intermittent transmission pattern, the timings indicated by the transmission pulse numbers 3, 6, 9, 12, and 15 in the reference transmission pattern are the omission timings (time periods during which the ultrasound pulses are thinned out). The broken line circular mark indicating the omission timing is marked with a reference numeral "B" indicating that the ultrasound pulses in the B-mode operation are transmitted and the reflected waves are received.

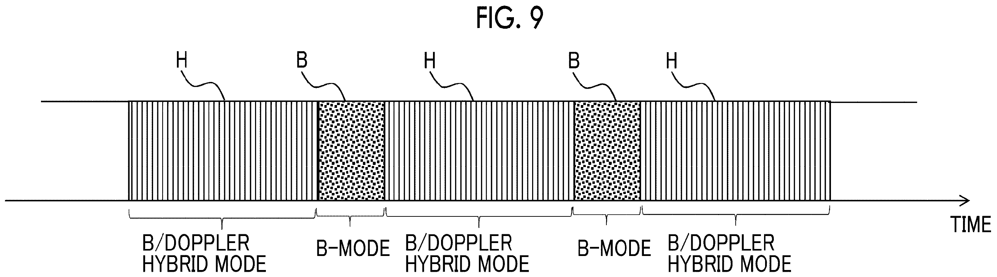

In such an operation of the B/Doppler hybrid mode, the ultrasound diagnostic apparatus 100 may generate the B-mode image data at the omission timing in a time division manner. In addition, as shown in FIG. 9, the ultrasound diagnostic apparatus 100 may alternately execute an operation H of the B/Doppler hybrid mode and an operation B of the B-mode in a time division manner. The B-mode image data may be generated sequentially over time by the time-division operation of the B-mode executed in the operation H of the B/Doppler hybrid mode and the operation B of the B-mode following the operation H of the B/Doppler hybrid mode.

According to such processing, the frame rate is improved by the intermittent transmission operation. In addition, a timing at which the B-mode image data is generated and a timing at which the Doppler measurement information is generated are brought closer together for a state of the subject 18 at a certain point in time.

(4-2) Adjacent Transmission and Reception Beams based on Complementary Intermittent Transmission Pattern

In a case where a plurality of transmission and reception beams are formed in the ultrasound probe 14, two complementary intermittent transmission patterns may be used for two adjacent transmission and reception beams. Here, the two complementary intermittent transmission patterns refer to two intermittent transmission patterns that are in a relationship in which a timing at which the ultrasound pulses are transmitted and received in one intermittent transmission pattern is an omission timing in the other intermittent transmission pattern.

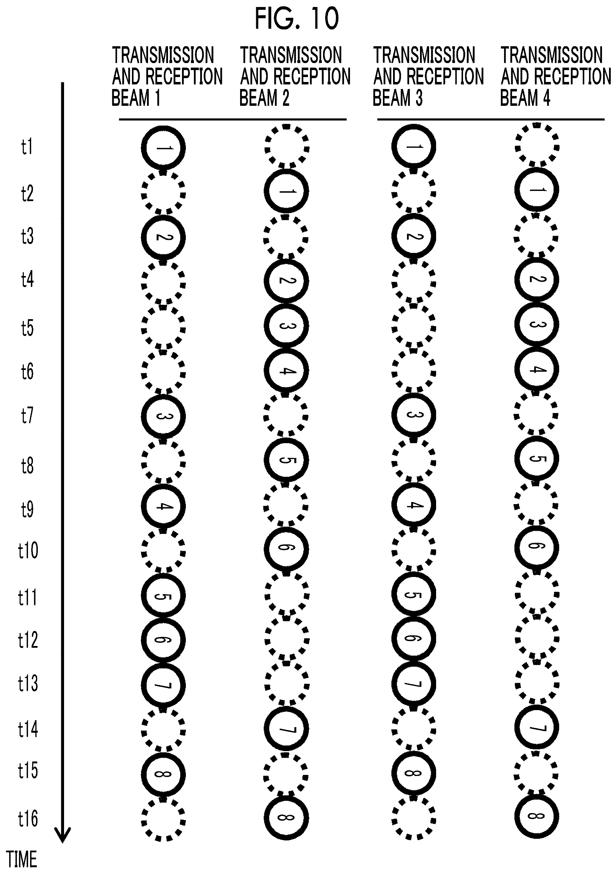

FIG. 10 shows an example in which two complementary intermittent transmission patterns are used for each of a pair of adjacent first and second transmission and reception beams, a pair of adjacent second and third transmission and reception beams, and a pair of adjacent third and fourth transmission and reception beams. In the example shown in FIG. 10, transmission timings t1, t3, t7, t9, t11, t12, t13, and t15 of one intermittent transmission pattern are omission timings of the other intermittent transmission pattern. In addition, transmission timings t2, t4, t5, t6, t8, t10, t14, and t16 of the other intermittent transmission pattern are omission timings of the one intermittent transmission pattern.

According to such processing, the frame rate is improved by the intermittent transmission operation. In addition, since interference between the adjacent transmission and reception beams is suppressed, the number of the transmission and reception beams per unit area may be increased. As a result, the image quality of the B-mode image and the color Doppler image is improved.

In the embodiment according to the present disclosure, each processing is executed by any computer. In addition, any computer may execute these processes using a processor as hardware, a program as software, or a combination thereof. In that case, the processor is configured to execute various processes in the present embodiment in cooperation with the program, and can function as each unit or each means in the present embodiment. In addition, the order in which the processes are executed by the processor is not limited to the order described above and may be changed as appropriate. Any computer may be a general-purpose computer, a computer for a specific use, a workstation, or another system capable of executing each processing.

The processor may be configured by one or more pieces of hardware, and the type of hardware is not limited. For example, the processor can be configured by hardware such as a central processing unit (CPU), a micro processing unit (MPU), a programmable logic device such as a field-programmable gate array (FPGA), a dedicated circuit for executing specific processing such as an application-specific integrated circuit (ASIC), a graphics processing unit

(GPU), or a neural processing unit (NPU). In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any embodiment, the order of each processing executed by the processor is not limited to the order described in the specification of the present application and may be changed as appropriate. The hardware is configured by an electric circuit (circuitry) in which circuit elements such as semiconductor elements are combined.

Further, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

The present disclosure can also be applied to a program and a program product. The transmission pattern determination program according to the above-described embodiment of the present disclosure may be provided by being stored in a storage medium such as a memory card, a USB memory, or a CD-ROM.

### Explanation of References

10: beam controller
12: transmission unit
14: ultrasound probe
16: transducer element
18: subject
20: reception unit
22: controller
24: operation unit
26: information processing unit
28: phasing addition unit
30: B-mode image generation unit
32: image processing unit
34: quadrature detection unit
36: interpolation processing unit
38: wall filter
40: Doppler measurement unit
42: operation setting unit
44: display unit

## Claims

1. An ultrasound diagnostic apparatus comprising:
a transmission unit that transmits a plurality of ultrasound pulses by using an ultrasound probe;
a reception unit that receives, by using the ultrasound probe, reflected waves generated a plurality of times from a measurement object; and
a processor configured to control the transmission unit and the reception unit,
wherein the processor is configured to
generate a plurality of received Doppler signal values from a plurality of received pulse signals output from the reception unit in response to the reflected waves generated a plurality of times from the measurement object and to obtain Doppler measurement information based on each of the received Doppler signal values, and
cause the transmission unit to execute intermittent transmission in which the ultrasound pulses are transmitted by omitting some of the ultrasound pulses based on an intermittent transmission pattern with respect to reference transmission in which the ultrasound pulses are transmitted a plurality of times at equal time intervals, and
the intermittent transmission pattern is a transmission pattern that is selected from any of a plurality of different intermittent transmission pattern candidates based on error evaluation information in a case where a step including obtaining reference measurement information for the reference transmission, obtaining intermittent measurement information for the intermittent transmission, and obtaining the error evaluation information is executed for the plurality of different intermittent transmission pattern candidates, the reference measurement information indicating a situation in which the Doppler measurement information is obtained, the intermittent measurement information indicating a situation in which the Doppler measurement information is obtained, and the error evaluation information representing a difference between the reference measurement information and the intermittent measurement information.

2. The ultrasound diagnostic apparatus according to claim 1,
wherein each of the reference measurement information and the intermittent measurement information includes a Doppler shift frequency, and
the error evaluation information includes a value indicating a difference between the Doppler shift frequency included in the reference measurement information and the Doppler shift frequency included in the intermittent measurement information.

3. The ultrasound diagnostic apparatus according to claim 1 or 2,
wherein the processor is configured to
perform filter processing on the plurality of received Doppler signal values in accordance with the number of the received Doppler signal values, and
generate the Doppler measurement information based on the plurality of received Doppler signal values on which the filter processing is performed,
each of the reference measurement information and the intermittent measurement information includes information indicating a filter characteristic according to the number of the received Doppler signal values, and
the error evaluation information includes a value indicating a difference between the filter characteristic included in the reference measurement information and the filter characteristic included in the intermittent measurement information.

4. The ultrasound diagnostic apparatus according to any one of claims 1 to 3,
wherein each of the reference measurement information and the intermittent measurement information includes aliasing characteristic information regarding a Doppler shift frequency, and
the error evaluation information includes information indicating a difference between the aliasing characteristic information included in the reference measurement information and the aliasing characteristic information included in the intermittent measurement information.

5. The ultrasound diagnostic apparatus according to any one of claims 1 to 4,
wherein the processor is configured to generate the plurality of received Doppler signal values from a plurality of received pulse signals obtained in response to the intermittent transmission and to obtain the Doppler measurement information based on a plurality of interpolated received Doppler signal values obtained by performing interpolation processing on the plurality of received Doppler signal values to interpolate signal values omitted due to the intermittent transmission.

6. The ultrasound diagnostic apparatus according to claim 5,
wherein the interpolation processing includes processing of interpolating the signal values omitted due to the intermittent transmission based on an interpolation function using an interpolation coefficient obtained in advance for the intermittent transmission pattern.

7. The ultrasound diagnostic apparatus according to any one of claims 1 to 6,
wherein the processor is configured to
control the transmission unit and the reception unit to operate in a B-mode in a time period in which the transmission of the ultrasound pulses is thinned out during the intermittent transmission, and
generate B-mode image data based on a signal output from the reception unit in the operation of the B-mode.

8. The ultrasound diagnostic apparatus according to any one of claims 1 to 7,
wherein the processor is configured to
control the transmission unit and the reception unit so that a plurality of transmission and reception beams are formed, and
cause the transmission unit to execute the intermittent transmission based on two complementary intermittent transmission patterns for two adjacent transmission and reception beams.

9. A transmission pattern determination method for obtaining an intermittent transmission pattern in an ultrasound diagnostic apparatus, the ultrasound diagnostic apparatus including
a transmission unit that transmits a plurality of ultrasound pulses by using an ultrasound probe,
a reception unit that receives, by using the ultrasound probe, reflected waves generated a plurality of times from a measurement object, and
a processor configured to control the transmission unit and the reception unit,
in which the processor is configured to
generate a plurality of received Doppler signal values from a plurality of received pulse signals output from the reception unit in response to the reflected waves generated a plurality of times from the measurement object and to obtain Doppler measurement information based on each of the received Doppler signal values, and
cause the transmission unit to execute intermittent transmission in which the ultrasound pulses are transmitted by omitting some of the ultrasound pulses based on the intermittent transmission pattern with respect to reference transmission in which the ultrasound pulses are transmitted a plurality of times at equal time intervals,
the transmission pattern determination method comprising:
executing, for a plurality of different intermittent transmission pattern candidates, a step including: obtaining reference measurement information for the reference transmission, the reference measurement information indicating a situation in which the Doppler measurement information is obtained; obtaining intermittent measurement information for the intermittent transmission, the intermittent measurement information indicating a situation in which the Doppler measurement information is obtained; and obtaining error evaluation information representing a difference between the reference measurement information and the intermittent measurement information; and
selecting any of the plurality of different intermittent transmission pattern candidates as the intermittent transmission pattern, based on the error evaluation information.

10. The transmission pattern determination method according to claim 9,
wherein each of the reference measurement information and the intermittent measurement information includes a Doppler shift frequency, and
the error evaluation information includes a value indicating a difference between the Doppler shift frequency included in the reference measurement information and the Doppler shift frequency included in the intermittent measurement information.

11. The transmission pattern determination method according to claim 9 or 10,
wherein the processor is configured to
perform filter processing on the plurality of received Doppler signal values in accordance with the number of the received Doppler signal values, and
generate the Doppler measurement information based on the plurality of received Doppler signal values on which the filter processing is performed,
each of the reference measurement information and the intermittent measurement information includes information indicating a filter characteristic according to the number of the received Doppler signal values, and
the error evaluation information includes a value indicating a difference between the filter characteristic included in the reference measurement information and the filter characteristic included in the intermittent measurement information.

12. The transmission pattern determination method according to any one of claims 9 to 11,
wherein each of the reference measurement information and the intermittent measurement information includes aliasing characteristic information regarding a Doppler shift frequency, and
the error evaluation information includes information indicating a difference between the aliasing characteristic information included in the reference measurement information and the aliasing characteristic information included in the intermittent measurement information.

13. A transmission pattern determination program for obtaining an intermittent transmission pattern in an ultrasound diagnostic apparatus, the ultrasound diagnostic apparatus including
a transmission unit that transmits a plurality of ultrasound pulses by using an ultrasound probe,
a reception unit that receives, by using the ultrasound probe, reflected waves generated a plurality of times from a measurement object, and
a processor configured to control the transmission unit and the reception unit,
in which the processor is configured to
generate a plurality of received Doppler signal values from a plurality of received pulse signals output from the reception unit in response to the reflected waves generated a plurality of times from the measurement object and to obtain Doppler measurement information based on each of the received Doppler signal values, and
cause the transmission unit to execute intermittent transmission in which the ultrasound pulses are transmitted by omitting some of the ultrasound pulses based on the intermittent transmission pattern with respect to reference transmission in which the ultrasound pulses are transmitted a plurality of times at equal time intervals,
the transmission pattern determination program being read into a computer and causing the computer to execute a process comprising:
executing, for a plurality of different intermittent transmission pattern candidates, a process including: obtaining reference measurement information for the reference transmission, the reference measurement information indicating a situation in which the Doppler measurement information is obtained; obtaining intermittent measurement information for the intermittent transmission, the intermittent measurement information indicating a situation in which the Doppler measurement information is obtained; and obtaining error evaluation information representing a difference between the reference measurement information and the intermittent measurement information; and
selecting any of the plurality of different intermittent transmission pattern candidates as the intermittent transmission pattern, based on the error evaluation information.
